# EUROPÄISCHE PATENTSCHRIFT

(11) **EP 3 522 806 B1**
(45) Veröffentlichungstag und Bekanntmachung des Hinweises auf die Patenterteilung: **20.01.2021**
(21) Anmeldenummer: 17783452.0
(22) Anmeldetag: 06.10.2017
(51) Int. Cl.: A61B 17/86

(54) **SCHRAUBE MIT SCHRAUBENANTRIEB MIT INTEGRIERTER DREHMOMENTSICHERUNG**
SCREW WITH SCREW DRIVE HAVING INTEGRATED TORQUE SECURITY
VIS AVEC EMPREINTE DE VIS À LIMITATION DE COUPLE INTÉGRÉE

(30) Priorität: 10.10.2016 DE 102016119234
(43) Veröffentlichungstag der Anmeldung: 14.08.2019
(73) Patentinhaber: Syntellix AG, 30159 Hannover (DE)
(72) Erfinder: SEITZ, Jan Marten, 30159 Hannover (DE); URBAN, Ute, 30159 Hannover (DE); HEINEMANN, Nils, 30159 Hannover (DE)
(74) Vertreter: Köllner, Malte
(86) Internationale Anmeldenummer: PCT/EP2017/075531
(87) Internationale Veröffentlichungsnummer: WO 2018/069190

(56) Entgegenhaltungen:
- US-A1- 2014 257 408
- US-A1- 2015 335 368
- US-A1- 2016 262 819

## Beschreibung

Die vorliegende Erfindung betrifft eine Schraube mit Schraubenantrieb.

Der Schraubenantrieb ist so gestaltet, dass eine mechanische Überbelastung der Schraube beim Eindrehen in das jeweilige Material, die zum Bruch des Schraubenkopfs, zum Abscheren der Schraube im Schaft oder zum Überdrehen und Ausreißen des Gewindes führen könnte, vermieden wird.

Beim Einsatz von Schrauben kann es leicht vorkommen, dass die Schraube durch eine zu hohe mechanische Belastung beschädigt wird, wodurch ein weiteres Eindrehen der Schraube in das jeweilige Material oder auch Entfernen der Schraube aus dem Material erschwert oder gar verhindert wird.

Schrauben werden in den unterschiedlichsten Gebieten eingesetzt.

Zur Osteosynthese bei Knochenbrüchen oder zur chirurgischen Korrektur von Fehlstellungen und Deformationen der Knochen, zum Beispiel bei Hallux Valgus (Ballenzehe), bei Hammerzehen, aber auch bei Brüchen von Speiche oder Elle, bei Frakturen im Bereich des Oberarms oder der Schulter sowie bei Brüchen der Fußwurzelknochen können heutzutage Schrauben aus biodegradierbaren Magnesiumlegierungen verwendet werden (Luthringer,J.C., Feyerabend F., Willumeit-Römer, R. - Magnesium Based Implants: A Mini Review, Magnesium Research 2014 (4) 142-154).

Diese speziellen Magnesium-Werkstoffe besitzen eine dem menschlichen Knochen vergleichbare Festigkeit, so dass sehr stabile und haltbare Verbindungen der zusammen zu fügenden Knochenfragmente möglich sind. Dadurch ist eine rasche und sichere Heilung des Knochenbruchs oder der Fehlstellung der Knochen gewährleistet. Gleichzeitig kann der Körper den in Form einer Schraube in den Knochen eingedrehten Magnesiumwerkstoff im Lauf etwa eines Jahres resorbieren und zu einem großen Teil sogar in körpereigene Knochensubstanz umwandeln. Nach dem Ausheilen der Fraktur muss die zur Fixierung der Knochenteile dienende Schraube nicht in einer zweiten Operation entfernt werden, wie es bei Verwendung nicht degradierbarer Schrauben aus Titan oder Edelstahl notwendig ist. Vielmehr wird die eingebrachte Magnesium-Schraube unter Bildung von Magnesium-Ionen allmählich aufgelöst. Die Magnesium-Ionen werden teilweise über die Nieren mit dem Urin ausgeschieden, teilweise auch im Knochen selbst in die sich bildende neue Knochensubstanz eingebaut.

Die Zug- und Bruchfestigkeit von biologisch abbaubaren Magnesiumlegierungen liegt über der Zug- und Bruchfestigkeit des menschlichen Knochens. In der Literatur findet man Angaben zur Zugfestigkeit menschlicher Knochen von 80 bis 150 MPa und Elastizitätsmodule von 10 bis 35 GPa (Wintermantel, E. und Ha, S.-W. in Biokompatible Werkstoffe und Bauweisen, Springer, Heidelberg - New York, 1996). Die Werte der Zugfestigkeit von biodegradierbaren Magnesiumlegierungen reichen von 100 MPa bis 400 MPa und von 40 bis 50 GPa für den Elastizitätsmodul. Von daher gesehen sind biodegradierbare Magnesiumwerkstoffe für die Osteosynthese also geradezu ideal geeignet, weil sie nahe an den Festigkeiten des Knochens liegen.

Biologisch abbaubare Magnesiumlegierungen sind daher auch vorteilhafter als polymer basierte biodegradierbare Werkstoffe, die diese Festigkeit nicht aufweisen. Vorteile im Vergleich mit Schrauben aus hochfesten Metallen wie Titan oder Edelstahl ergeben sich daraus, dass Implantate aus diesen Werkstoffen nach der Heilung häufig in einer zweiten Operation wieder entfernt werden müssen, was bei den biodegradierbaren Magnesiumwerkstoffen entfällt. Zudem beobachtet man bei Einsatz von Werkstoffen wie Stahl oder Titan, deren Festigkeit sehr weit über der des Knochens liegt, einen unerwünschten Stress Shielding Effekt, der die Heilung behindert und im ungünstigsten Fall sogar zu einem Knochenabbau in der Umgebung des Implantats führen kann (B. Daniels et al., Journal of Applied Biomaterials 1 / 1 (1990), S. 57-98).

Bei Operationen zur Osteosynthese eines gebrochenen Knochens oder nach der chirurgischen Korrektur deformierter Knochen wird der Chirurg die zusammen zu fügenden Teile des Knochens repositionieren und mit einer Schraube sicher fixieren. Die Schraube wird im Allgemeinen in einen in den Knochen vorgebohrten und ggf. mit einem Gewinde vorgeschnittenen Kanal eingedreht. Das Eindrehen einer sterilen biodegradierbaren Magnesiumschraube erfolgt mit einem speziellen sterilen Schraubendreher.

Beim Eindrehen der Schraube kann es zu folgenden Komplikationen kommen:
Zum einen kann die Schraube klemmen, etwa weil der vorgebohrte Kanal zu eng oder zu kurz ist, oder weil ein Gewinde nicht ordnungsgemäß vorgeschnitten wurde. Es besteht die Gefahr, dass der Chirurg die Magnesiumschraube am Kopf zu fest dreht und dabei den Schraubenkopf abbricht, abschert oder sogar einen Schaftbruch herbeiführt. Wenn die Schraube noch nicht vollständig eingedreht wurde, ist die Fixierung der Fraktur nicht gewährleistet. Die Schraube müsste wieder entfernt werden, was bei einem abgebrochenen Schraubenkopf schwierig ist und nur mit speziellen Werkzeugen erfolgen kann. Unter Umständen kann ein zusätzlicher chirurgischer Eingriff und in einzelnen Fällen auch ein Entfernen durch Ausbohren des Implantats notwendig werden. Die genannten Prozeduren sind zeitaufwändig, die Operation würde sich in jedem Fall erheblich in die Länge ziehen.

Zudem kann es vorkommen, dass der Chirurg den Bruch zwar ideal fixiert hat und die Magnesiumschraube vollständig eingedreht wurde. Dann ist die Schraube so fest anzuziehen, dass ein optimaler Kraftschluss zwischen den Knochenbruchstücken entsteht. Dabei besteht die Gefahr, dass die Schraube im Gewinde überdreht wird und ausreißt, wenn ihre Haltekraft überbelastet wird. Der Kraftschluss würde zusammen brechen, die Schraube würde nur noch eine lockere Verbindung der Knochenfragmente herstellen. In dieser Situation muss der Chirurg anhand seines Gefühls entscheiden, ob die Schraube optimal sitzt oder ob sie bereits überdreht ist. Zudem kann durch übermäßiges Anziehen der Schraube auch die bereits zuvor geschilderte Komplikation eintreten, etwa der Fall, dass der Schraubenkopf abreißt.

In all den genannten Situationen kann es zu einer kosten- und zeitaufwändigen Verlängerung der Operationszeit kommen.

Auch in anderen Gebieten, in denen Schrauben eingesetzt werden, wie u.a. elektronische und feinmechanische Bauteile, Uhren, Spielzeuge, Möbel, elektrische Haushaltsgeräte, Sportgeräte, kann es vorkommen, dass der Schraubenantrieb durch eine zu hohe mechanische Belastung beschädigt wird, wodurch ein weiteres Eindrehen oder auch Entfernen der Schraube erschwert oder gar verhindert wird.

Aus der US 2016/0262819 A1, US 2015/0335368 A1 und US 2014/0257408 A1 sind beispielsweise Schrauben mit zwei Schraubenantrieben bekannt. Ein Antrieb ist dabei ein Innen-Mehrkant-Antrieb, und der zweite Antrieb ein Innen-Mehrrund -Antrieb in Form eines mehrstrahligen Sterns, bei dem die Strahlen in Form von abgerundeten Zapfen oder anderen Formen der Zapfen geformt sind.

Die Aufgabe der vorliegenden Erfindung war es, einen Schraubenantrieb bereit zu stellen, der es ermöglicht, die Schraube trotz Beschädigung des Antriebs weiter in das Material Eindrehen oder auch Entfernen zu können.

Gelöst wird die Aufgabe durch die erfindungsgemäße Schraube nach Anspruch 1.

Bevorzugt ist ein Schraubenantrieb mit integrierter Drehmomentsicherung, wobei in einen Schraubenkopf zwei Schraubenantriebe eingearbeitet sind, wobei ein Antrieb ein Innen-Mehrkant-Antrieb ist, und der zweite Antrieb ein Innen-Mehrrund -Antrieb ist.

In den Schraubenkopf sind dafür zwei kombinierte Schraubenantriebe eingearbeitet, wobei ein Antrieb ein Innen-Mehrkant-Antrieb ist, der ein Zurückdrehen der Schraube gestattet, und der zweite Antrieb ein Innen-Mehrrund-Antrieb ist, der so ausgestaltet ist, dass bei Überschreiten eines auf die Konstruktion der Schraube abgestimmten Drehmoments die den Antrieb ermöglichenden Zapfen abbrechen, abscheren oder flach gedrückt werden, so dass eine Beschädigung des Gewindes, des Schraubenschafts oder das Abreißen des Schraubenkopfs verhindert wird.

Der Schraubenantrieb weist somit eine konstruktive integrierte Drehmomentsicherung auf, die ein Abbrechen des Schraubenkopfes, ein Abscheren des Schraubenkopfs oder des Schaftes, ein Ausreißen des Gewindes oder ein Überdrehen der Schraube im Knochenkanal sowie weitere durch Überbelastung der Schraube mögliche Beschädigungen verhindert und ein einfaches Entfernen der Schraube nach eventueller Überbelastung ermöglicht.

Es ist weiterhin Gegenstand der vorliegenden Erfindung, dass der Innen-Mehrrund -Antrieb so auf den Innen-Mehrkant-Antrieb angesetzt ist, dass nach dem Abbrechen der Zapfen des Innen-Mehrrund-Antriebs aufgrund des Überschreitens des maximalen Drehmoments der Mehrkant-Innen-Antrieb erhalten bleibt. Unter Abbrechen der Zapfen im Sinne der vorliegenden Erfindung ist dabei jegliches Abbrechen oder Abscheren der Zapfen aufgrund des Überschreitens des maximalen Drehmoments zu verstehen.

Es ist dabei vorteilhaft, wenn die Zapfen des Innen-Mehrrund Antriebes in einer Ebene mit dem Innen-Mehrkant-Antrieb liegen. Die Zapfen des Innen-Mehrrund Antriebes liegen dann unmittelbar auf den Kanten des Innen-Mehrkant-Antriebs und mit diesen in einer Ebene.

Das Antriebswerkzeug, ein spezieller Innen-Mehrrund Schraubendreher, rutscht dann im Schraubenkopf durch und kann keine Kraft bzw. kein Drehmoment mehr auf die Schraube übertragen. Die Höhe der Zapfen, die nach dem Abbrechen zurück bleiben liegt im Allgemeinen bei Bruchteilen von Millimetern. Die Zapfenreste stellen keine Hindernisse für den Einsatz eines Innen-Mehrkant Schraubendrehers dar, mit dem die Schraube wieder heraus gedreht werden kann.

In einer weiteren vorteilhaften Ausgestaltungsform ist der Innen-Mehrkant-Antrieb tiefer in der Schraube gelegen als der Innen- Mehrrund-Antrieb. Die Zapfen des Innen-Mehrrund Antriebes liegen dann nicht mit den Kanten des Innen-Mehrkant-Antrieb in einer Ebene, sondern darüber. Auch kann es bevorzugt sein, dass der Innen-Mehrkant-Antrieb einen geringeren Durchmesser aufweist als der Innen-Mehrrund-Antrieb. Als Durchmesser für den Innen-Mehrrund-Antrieb ist dabei der Kreis zu verstehen, der die Zapfen innen an ihrer Basis tangiert (H). Als Durchmesser für den Innen-Mehrkant-Antrieb ist dabei der Kreis zu verstehen, der alle Kanten tangiert (B).Der erfindungsgemäße Schraubenantrieb ist auf dem Gebiet der Osteosynthese insbesondere für biodegradierbare Magnesiumschrauben geeignet, jedoch nicht auf diesen Einsatz beschränkt. Durch den erfindungsgemäßen Schraubenantrieb im Kopf einer Schraube, vorzugsweise einer biodegradierbaren Magnesiumschraube, wird die durch ein Werkzeug aufgebrachte Drehbewegung sicher in die Schraube zu übertragen. Auch sind andere biodegradierbare Materialien, wie Poylmere, für diesen Antrieb und die entsprechende Schraube geeignet.

Der Schraubenantrieb ist jedoch nicht auf das Gebiet der Osteosynthese beschränkt. Jegliche andere Einsatzgebiete und Materialien von Schrauben, die ein Abbrechen der Zapfen erlauben, wie Metalle oder Holz, sind mit umfasst. In einer bevorzugten Ausführungsform besteht der erfindungsgemäße Schraubenantrieb aus mindestens einem Polymer oder aus Holz oder aus Keramik oder aus Composite oder aus mindestens einem Metall.

Die beim Ansprechen der Drehmomentsicherung abgescherten Zapfen oder Bruchstücke davon stellen auf dem Gebiet der Osteosynthese keine Gefahr für den Patienten dar, da sie aus einem biodegradierbaren Material bestehen. Sofern Abrieb oder Fragmente nicht ohnehin heraus gespült, gesammelt und entfernt werden, können sie im Bereich der Operationswunde bedenkenlos verbleiben, da sie, wie das Schraubenimplantat selbst, vom Körper restlos resorbiert bzw. abgebaut werden. Die biodegradierbaren Magnesiumlegierungen stellen keine Belastung für das physiologische System dar. Der Vorteil der erfindungsgemäßen Ausführung des Schraubenantriebs besteht darin, dass bei Komplikationen während einer Operation sehr rasch und sicher, mit einfachsten Mitteln, die Schrauben wieder entfernt und geeignete Korrekturen vorgenommen werden können.

Unter Innen-Mehrkant-Antrieb ist im Sinne der vorliegenden Erfindung vorzugsweise ein Innensechskant Antrieb, beispielsweise ein Inbus, zu verstehen. Es ist dabei vorgesehen, dass der Innen-Mehrkant-Antrieb als Innen-Sechskant- Antrieb oder als Innen-Fünfkant-Antrieb oder als Innen-Vierkant-Antrieb oder als Innen-Dreikant-Antrieb oder als Innen-Achtkant-Antrieb ausgebildet ist. Es können auch weitere Innen-Mehrkant-Antriebe mit einer anderen Anzahl an Kanten vorteilhaft sein.

Unter Innen-Mehrrund-Antrieb ist im Sinne der vorliegenden Erfindung ein Schraubenantrieb in Form eines mehrstrahligen Sterns zu verstehen, bei dem die Strahlen (Zapfen) abgerundet sind. Es kann sich dabei um einen sechsstrahligen Stern (Innen-Sechsrund, Torx®) oder auch um einen Innen-Fünfrund-Antrieb oder Innen-Vierrund-Antrieb oder Innen-Dreirund-Antrieb oder Innen-Achtrund-Antrieb handeln. Es können auch weitere Innen-Mehrrund-Antriebe mit einer anderen Anzahl an Zapfen und anderen Formen der Zapfenvorteilhaft sein.

Es können anstatt der abgerundeten Zapfen auch dreieckige Zapfen, viereckige Zapfen- mit oder ohne Fasen - halbrunde oder auch ovale Zapfen als Überdrehsicherung verwendet werden.

Um sehr niedrige Drehmomente abzusichern, können einzelne Zapfen weg gelassen werden. So können statt der 6 Zapfen auf einem Innensechskant Profil auch nur 3 Zapfen verwendet werden.

Zudem können die Zapfen noch gezielt geschwächt werden, um das maximale Drehmoment besonders fein und präzise an die Gesamtkonstruktion der Schraube anzupassen. Dies wird durch Bohrungen erreicht, die in die Zapfen eingebracht werden. Es ist daher weiterhin bevorzugt, dass die drehmomentsichernden Zapfen des Innen-Mehrrund-Antriebs durch eine Bohrung oder mehrere Bohrungen gezielt so geschwächt werden, dass auch sehr geringe Drehmomente zu einem Ansprechen der Schraubensicherung durch Abbrechen der Zapfen führen.

Gemäß einem weiteren Aspekt der vorliegenden Erfindung beträgt
der Durchmesser des Innensechskant Antriebs (B) 1-20 mm, vorzugsweise 3 mm, die Tiefe des Schraubenantriebs (C) 0,5-6 mm, vorzugsweise 2,5 mm,
die Dicke der drehmomentsichernden Zapfen an der Basis (F) 0,1-4 mm,
vorzugsweise 0,5 mm,
die Höhe der drehmomentsichernden Zapfen (G) 0,1-3 mm, vorzugsweise 0,5 mm,
der Rest Höhe der Zapfen nach Abbrechen oder Abscheren (G*) 0,01-1 mm,
vorzugsweise 0,1 mm und der Durchmesser der Bohrung in den Zapfen zur
Schwächung des maximalen Drehmoments (2R) 0,06-1,6 mm,
vorzugsweise 0,2 mm.

In einer bevorzugten Ausführungsform besteht der Schraubenantrieb aus einer Magnesiumlegierung mit
einem Seltenerdmetallanteil von 1,5 bis 5 Gew. %, davon 1,5 bis 2,5 Gew. % Neodym, einem Yttriumanteil von 1,5 bis 5 Gew. %, einem Zirkoniumanteil von 0,1 bis 2,5 Gew. %, einem Zinkanteil von 0,01 bis 1 Gew.% sowie unvermeidbaren Verunreinigungen, wobei der Gesamtgehalt an möglichen Verunreinigungen unterhalb von 1 Gew. % liegt und der Aluminiumanteil kleiner als 0,5 Gew. % ist, und der Rest zu 100 Gew. % Magnesium enthält.

Zumeist besteht der Schraubenantrieb aus etwa 80 Gew. % Magnesium, meistens etwa 90 Gew. % Magnesium und den Legierungselementen Calcium, Lithium, Zink, Scandium, Yttrium, Lanthan und Metallen der Seltenen Erden, insbesondere Neodym, Cer, Dysprosium und anderen SE-Metallen. Auch können Titan, Zirkonium, Mangan oder Silber in kleineren Mengen zu legiert sein. Elemente wie Kupfer, Aluminium, Eisen, Nickel, und Phosphor sind demgegenüber als eher schädliche Verunreinigungen zu betrachten und sollten in dentuelln biologisch abbaubaren Legierungen vorteilhafterweise nur in Konzentrationen unter 0,1 Gew. % vorhanden sein. Diese Legierung weist eine Zugfestigkeit von ca. 300 MPa auf.

Die Herstellung des drehmomentgesicherten Schraubenantriebs erfolgt vorzugsweise durch filigranes Ausfräsen aus einem Schrauben-Rohling. Alternativ kann der Schraubenkopf mit dem erfindungsgemäßen drehmomentgesicherten Antrieb auch durch mechanisches Pressen eines Rohlings in einer entsprechenden Form oder durch das Verfahren des Druckgießens gefertigt werden. Bei einigen Werkstoffen, z.B. bei Kunststoffen oder polymeren Werkstoffen kann auch das Verfahren des 3-D-Drucks angewendet werden.

Es ist weiterhin bevorzugt, dass die Drehmomente, bei denen die Drehmomentsicherung des erfindungsgemäßen Schraubenantriebs durch Abscheren der Sicherungszapfen eintritt, bei einer Schraube mit einem
Durchmesser des Schraubenkopfs (A) von 4 mm, einem
Durchmesser des Innen-Mehrkant Antriebs (B) von 2,12 mm, einer
Tiefe des Schraubenantriebs (C) von 1,2 mm, einem
Durchmesser des Schraubenschafts / Gewinde-Durchmesser (D) von 2 mm, einer Gesamtlänge der Schraube (E) von 6-20 mm, einer
Dicke der drehmomentsichernden Zapfen an der Basis (F) von 0,322 mm, und einer Höhe der drehmomentsichernden Zapfen (G) von 0,211 mm,
0,28 Nm beträgt.

Auch ist es bevorzugt, dass die Drehmomente, bei denen die Drehmomentsicherung des erfindungsgemäßen Schraubenantriebs durch Abscheren der Sicherungszapfen eintritt, bei einer Schraube mit einem
Durchmesser des Schraubenkopfs (A) von 5 mm, einem
Durchmesser des Innen-Mehrkant Antriebs (B) von 2,06 mm, einer
Tiefe des Schraubenantriebs (C) von 1,6 mm, einem
Durchmesser des Schraubenschafts / Gewinde-Durchmesser (D) von 2,7 mm,
einer
Gesamtlänge der Schraube (E) von 6-30 mm, einer
Dicke der drehmomentsichernden Zapfen an der Basis (F) von 0,4 mm, und
einer Höhe der drehmomentsichernden Zapfen (G) von 0,3 mm,
0,67 Nm beträgt.

Weiterhin ist es vorteilhaft, dass die Drehmomente, bei denen die Drehmomentsicherung des Schraubenantriebs durch Abscheren der Sicherungszapfen eintritt, bei einer Schraube mit einem
Durchmesser des Schraubenkopfs (A) von 6 mm, einem
Durchmesser des Innen-Mehrkant Antriebs (B) von 2,62 mm, einer
Tiefe des Schraubenantriebs (C) von 1,8 mm, einem
Durchmesser des Schraubenschafts / Gewinde-Durchmesser (D) von 3,5 mm,
einerGesamtlänge der Schraube (E) von 8-40 mm, einer
Dicke der drehmomentsichernden Zapfen an der Basis (F) von 0,4 mm, und
einer Höhe der drehmomentsichernden Zapfen (G) von 0,27 mm,
1,42 Nm beträgt.

Durch den Schraubenantrieb im Kopf der Schraube wird die durch ein Werkzeug aufgebrachte Drehbewegung sicher in die Schraube zu übertragen.

Dieser Schraubenantrieb ist auf dem Gebiet der Osteosynthese insbesondere für biodegradierbare Magnesiumschrauben geeignet, jedoch nicht auf diesen Einsatz beschränkt. Auch sind andere biodegradierbare Materialien, wie Poylmere, für diesen Antrieb und die entsprechende Schraube geeignet.

Der Schraubenantrieb und die erfindungsgemäße Schraube sind nicht auf das Gebiet der Osteosynthese beschränkt. Jegliche andere Einsatzgebiete und Materialien von Schrauben, die ein Abbrechen der Zapfen erlauben, wie Metalle oder Holz, sind mit umfasst.

In einer bevorzugten Ausführungsform bestehen der Schraubenantrieb und/oder die Schraube aus mindestens einem Polymer oder aus Holz oder aus Keramik oder aus Composite oder aus mindestens einem Metall. Schraube und Schraubenantrieb können dabei auch aus unterschiedlichen Materialien bestehen, müssen demnach nicht aus demselben Material gefertigt sein.

In einer bevorzugten Ausführungsform besteht eine biodegradierbare Magnesiumschraube und der Schraubenantrieb aus einer Magnesiumlegierung mit
einem Seltenerdmetallanteil von 1,5 bis 5 Gew. %, davon 1,5 bis 2,5 Gew. % Neodym, einem Yttriumanteil von 1,5 bis 5 Gew. %, einem Zirkoniumanteil von 0,1 bis 2,5 Gew. %, einem Zinkanteil von 0,01 bis 1 Gew. % sowie unvermeidbaren Verunreinigungen, wobei der Gesamtgehalt an möglichen Verunreinigungen unterhalb von 1 Gew. % liegt und der Aluminiumanteil kleiner als 0,5 Gew. % ist, und der Rest zu 100 Gew. % Magnesium enthält.

Zumeist bestehen die Schraube und der Schraubenantrieb aus etwa 80 Gew. % Magnesium, meistens etwa 90 Gew. % Magnesium und den Legierungselementen Calcium, Lithium, Zink, Scandium, Yttrium, Lanthan und Metallen der Seltenen Erden, insbesondere Neodym, Cer, Dysprosium und anderen SE-Metallen. Auch können Titan, Zirkonium, Mangan oder Silber in kleineren Mengen zu legiert sein. Elemente wie Kupfer, Aluminium, Eisen, Nickel, und Phosphor sind demgegenüber als eher schädliche Verunreinigungen zu betrachten und sollten in den biologisch abbaubaren Legierungen nur in Konzentrationen unter 0,1 Gew. % vorhanden sein.

Diese Legierung weist eine Zugfestigkeit von ca. 300 MPa auf.

Die Herstellung des drehmomentgesicherten Schraubenantriebs erfolgt vorzugsweise durch filigranes Ausfräsen aus einem Schrauben-Rohling. Alternativ kann der Schraubenkopf mit dem erfindungsgemäßen drehmomentgesicherten Antrieb auch durch mechanisches Pressen eines Rohlings in einer entsprechenden Form oder durch das Verfahren des Druckgießens gefertigt werden. In einer vorteilhaften Ausgestaltung ist auch das Verfahren des 3-D Druck für passende Werkstoffe vorgesehen.

Es ist dabei vorteilhaft, wenn die Zapfen des Innen-Mehrrund Antriebes in einer Ebene mit dem Innen-Mehrkant-Antrieb liegen. Die Zapfen des Innen-Mehrrund Antriebes liegen dann auf den Kanten des Innen-Mehrkant-Antriebs und mit diesen in einer Ebene.

In einer weiteren vorteilhaften Ausgestaltungsform ist der Innen-Mehrkant-Antrieb tiefer in der Schraube gelegen als der Innen- Mehrrund-Antrieb. Die Zapfen des Innen-Mehrrund Antriebes liegen dann nicht mit den Kanten des Innen-Mehrkant-Antrieb in einer Ebene, sondern darüber. Auch kann es bevorzugt sein, dass der Innen-Mehrkant-Antrieb einen geringeren Durchmesser aufweist als der Innen-Mehrrund-Antrieb. Als Durchmesser für den Innen-Mehrrund-Antrieb ist dabei der Kreis zu verstehen, der die Zapfen innen an ihrer Basis tangiert (H). Als Durchmesser für den Innen-Mehrkant-Antrieb ist dabei der Kreis zu verstehen, der alle Kanten tangiert (B).

Gemäß einem weiteren Aspekt der vorliegenden Erfindung beträgt der
Durchmesser des Schraubenkopfs (A) 2-30 mm, vorzugsweise 6 mm,
der Durchmesser des Innensechskant Antriebs (B) 1-20 mm, vorzugsweise 3 mm,
die Tiefe des Schraubenantriebs (C) 0,5-6 mm, vorzugsweise 2,5 mm,
der Durchmesser des Schraubenschafts / Gewinde-Durchmesser (D) 2-15 mm,
vorzugsweise 3,5 mm,
die Gesamtlänge der Schraube (E) 5-120 mm, vorzugsweise 30 mm,
die Dicke der drehmomentsichernden Zapfen an der Basis (F) 0,1-4 mm,
vorzugsweise 0,5 mm
die Höhe der drehmomentsichernden Zapfen (G) 0,1-3mm, vorzugsweise 0,5 mm,
der Rest Höhe der Zapfen nach Abbrechen oder Abscheren (G*) 0,01-1mm,
vorzugsweise 0,1 mm und der Durchmesser der Bohrung in den Zapfen zur
Schwächung des maximalen Drehmoments (2R) 0,06-1,6 mm,
vorzugsweise 0,2 mm.

Es ist weiterhin bevorzugt, dass die Drehmomente, bei denen die Drehmomentsicherung des Schraubenantriebs durch Abscheren der Sicherungszapfen eintritt, bei einer Schraube mit einem
Durchmesser des Schraubenkopfs (A) von 4 mm, einem
Durchmesser des Innen-Mehrkant Antriebs (B) von 2,12 mm, einer
Tiefe des Schraubenantriebs (C) von 1,2 mm, einem
Durchmesser des Schraubenschafts / Gewinde-Durchmesser (D) von 2 mm, einer Gesamtlänge der Schraube (E) von 6-20 mm, einer

Dicke der drehmomentsichernden Zapfen an der Basis (F) von 0,322 mm, und
einer Höhe der drehmomentsichernden Zapfen (G) von 0,211 mm
0,28 Nm beträgt.

Auch ist es bevorzugt, dass die Drehmomente, bei denen die Drehmomentsicherung des erfindungsgemäßen Schraubenantriebs durch Abscheren der Sicherungszapfen eintritt, bei einer Schraube mit einem
Durchmesser des Schraubenkopfs (A) von 5 mm, einem
Durchmesser des Innen-Mehrkant Antriebs (B) von 2,06 mm, einer
Tiefe des Schraubenantriebs (C) von 1,6 mm, einem
Durchmesser des Schraubenschafts / Gewinde-Durchmesser (D) von 2,7 mm,
einer
Gesamtlänge der Schraube (E) von 6-30 mm, einer
Dicke der drehmomentsichernden Zapfen an der Basis (F) von 0,4 mm, und
einer Höhe der drehmomentsichernden Zapfen (G) von 0,3 mm
0,67 Nm beträgt.

Weiterhin ist es vorteilhaft, dass die Drehmomente, bei denen die Drehmomentsicherung des erfindungsgemäßen Schraubenantriebs durch Abscheren der Sicherungszapfen eintritt, bei einer Schraube mit einem
Durchmesser des Schraubenkopfs (A) von 6 mm, einem
Durchmesser des Innen-Mehrkant Antriebs (B) von 2,62 mm, einer
Tiefe des Schraubenantriebs (C) von 1,8 mm, einem
Durchmesser des Schraubenschafts / Gewinde-Durchmesser (D) von 3,5 mm,
einer
Gesamtlänge der Schraube (E) von 8-40 mm, einer
Dicke der drehmomentsichernden Zapfen an der Basis (F) von 0,4 mm, und
einer Höhe der drehmomentsichernden Zapfen (G) von 0,27 mm
1,42 Nm beträgt.

Die vorliegende Erfindung wird anhand der folgenden Figuren näher erläutert.
- Figur 1: Längsschnitt einer Magnesiumschraube zur Osteosynthese
- Figur 2: Prinzip des erfindungsgemäßen Schraubenkopfs in einer Ausführung mit Innensechskant und sechs innen liegenden Zapfen, die das Drehmoment begrenzen
- Figur 3: Aufsicht auf einen erfindungsgemäßen Schraubenkopf gemäß Figur 2 nach Überschreiten des maximalen Drehmoments
- Figur 4: Aufsicht auf einen erfindungsgemäßen Schraubenkopf mit Innensechskant und sechs innen liegenden Zapfen, die das Drehmoment begrenzen
- Figur 5: Prinzip des erfindungsgemäßen Schraubenkopfs bei dem die drehmomentsichernden Zapfen in einen Innen-Vierkant eingearbeitet sind
- Figur 6: Aufsicht wie Figur 5, jedoch nach Abscheren der das maximale Drehmoment sichernden Zapfen
- Figur 7: Prinzip-Skizze eines drehmomentgesicherten Schraubenkopfs mit dreieckigen Zapfen
- Figur 8: Besonders vorteilhafter drehmomentgesicherter Schraubenkopf einer erfindungsgemäßen Magnesiumschraube mit sechs innen liegenden Zapfen, die das Drehmoment bei 1,42 Nm begrenzen
- Figur 9: Drehmomentgesicherter Schraubenkopf einer erfindungsgemäßen Magnesiumschraube mit sechs innen liegenden Zapfen, wobei Bohrungen mit Radius R in den Zapfen eine gezielte Schwächung bewirken zur Anpassung des Drehmoments an die konstruktive Auslegung der Schraube
- Figur 10: Ausgestaltungsform, bei der der Innen-Mehrkant-Antrieb tiefer in der Schraube gelegen als der Innen- Mehrrund-Antrieb

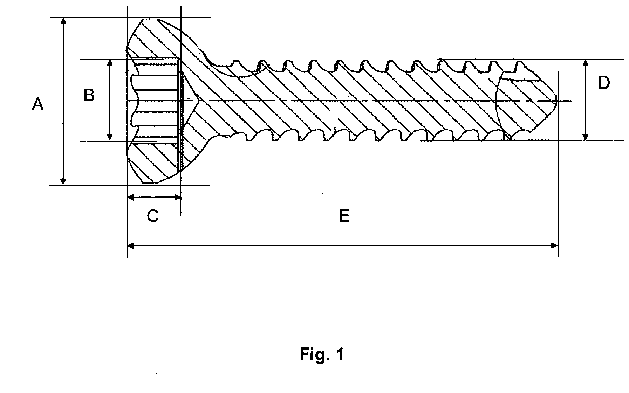

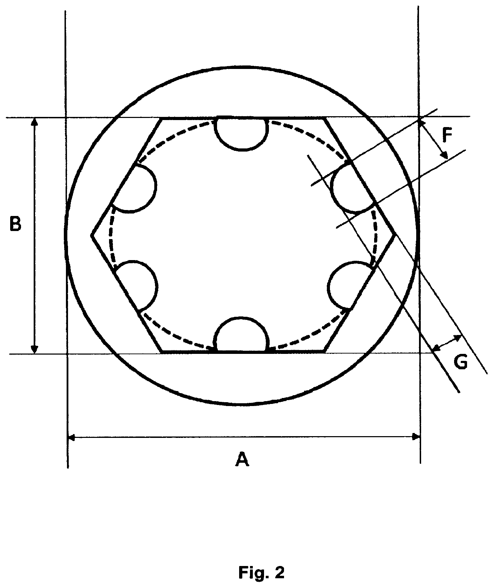

Konstruktionen zur Ausführung der Erfindung sind in den Figuren 1 bis 9 abgebildet. Diese sind zur Erläuterung auf das Beispiel der Osteosynthese bezogen, jedoch ist die vorliegende Erfindung nicht auf dieses Gebiet beschränkt. Figur 1 zeigt den typischen Längsschnitt durch eine erfindungsgemäße Magnesium-Schraube, wie sie bei Osteosynthesen verwendet wird. Verschiedene Varianten des erfindungsgemäßen Schraubenantriebs und der erfindungsgemäßen Schraube mit Schraubenantrieb zeigen die Figuren 2 bis 9.

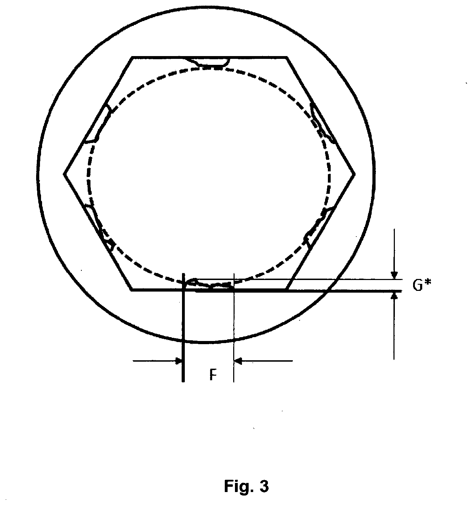

Das Prinzip der Erfindung in der Ausgestaltungsform, bei der die Zapfen des Innen-Mehrrund Antriebes in einer Ebene mit dem Innen-Mehrkant-Antrieb liegen, ist in Figur 2 und 3 dargestellt. Als Schraubenantrieb ist in den Kopf der aus einem biodegradierbaren Magnesiumwerkstoff bestehenden Schraube ein Innensechsrund-Antrieb eingearbeitet mit 3 bis 6, vorzugsweise 6 Zapfen, wobei die Zapfen auf den Kanten eines quasi dahinter liegenden Innensechskant-Antriebs (Sechskant-Inbus) aufgesetzt sind. F und G markieren dabei Breite und Dicke der Zapfen, A den Durchmesser des Schraubenkopfs und B den Durchmesser des Innensechskant-Profils, an das die Zapfen angesetzt sind.

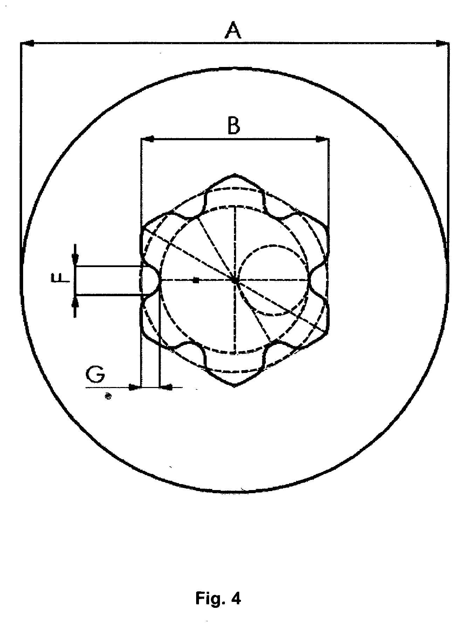

Den Antrieb einer Magnesium-Schraube, bei der die erfindungsgemäße Drehmomentsicherung in einer Torx-Ausführung gemäß DIN EN ISO 10664 auf Sechskant-Inbus ausgeführt ist, zeigt Figur 4.

Die Torx - Zapfen sind konstruktiv in ihren Abmessungen so ausgelegt, dass sie sich abscheren, abreißen oder seitlich weg drücken, wenn eine zu hohe Kraft auf sie einwirkt bzw. ein Drehmoment auf sie wirkt, das so groß ist, dass die Gefahr besteht, den gesamten Kopf der Magnesiumschraube abzubrechen oder abzuscheren - oder wenn ein Drehmoment auf den Schraubenkopf wirkt, das die Presskraft / Haltekraft der Schraube übersteigt.

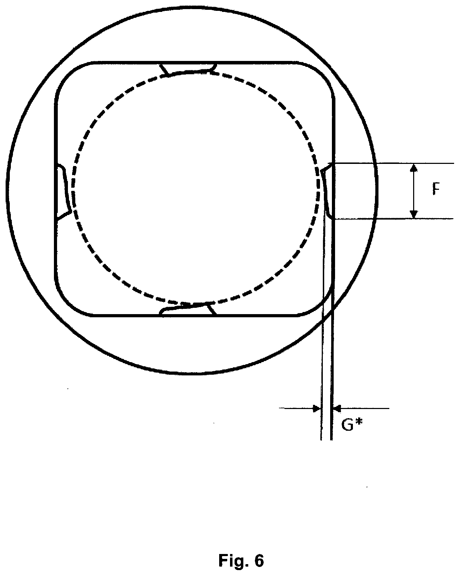

In diesem Fall brechen die Zapfen des Torx Antriebs ab oder werden seitlich weg gedrückt, noch bevor eine Beschädigung der Schraube oder des Gewindes erfolgt. Das Antriebswerkzeug, ein spezieller Torx Schraubendreher, rutscht dann im Schraubenkopf durch und kann keine Kraft bzw. kein Drehmoment mehr auf die Schraube übertragen. Die Konstruktion kann als eine "integrierte Drehmoment-Sicherung im Schraubenkopf" bezeichnet werden. Die Situation, die nach dem Abscheren der Torx - Zapfen auftritt, ist schematisch in Figur 3 und Figur 6 dargestellt. G* bezeichnet danach die Höhe der Zapfen, die nach dem Abbrechen zurück bleiben. Sie liegt im Allgemeinen bei Bruchteilen von Millimetern. Die Zapfenreste stellen keine Hindernisse für den Einsatz eines Inbus Schraubendrehers dar, mit dem die Schraube wieder heraus gedreht werden kann.

Ist der Fall eingetreten, dass die Drehmoment Sicherung angesprochen hat, die Torx Zapfen also ab geschert wurden, kann die Schraube mit dem Torx Dreher nicht mehr weiter gedreht werden. Sie kann aber über den integrierten Sechskantantrieb, auf den die Torx Zapfen aufgesetzt waren, und der bei diesem Vorgang erhalten bleibt, noch zurück gedreht und damit bei Bedarf leicht und sicher entfernt werden. Dies ist aus Figur 3 ebenfalls zu erkennen. Nach chirurgischer Korrektur des Knochenkanals kann dann eine neue, gegebenenfalls stärkere, kürzere oder insgesamt anders konstruierte biodegradierbare Schraube eingesetzt werden.

Die beim Ansprechen der Drehmomentsicherung abgescherten Zapfen oder Bruchstücke davon stellen keine Gefahr für den Patienten dar, da sie aus einem biodegradierbaren Material bestehen. Sofern Abrieb oder Fragmente nicht ohnehin heraus gespült, gesammelt und entfernt werden, können sie im Bereich der Operationswunde bedenkenlos verbleiben, da sie, wie das Schraubenimplantat selbst, vom Körper restlos resorbiert bzw. abgebaut werden. Die biodegradierbaren Magnesiumlegierungen stellen keine Belastung für das physiologische System dar. Der Vorteil der erfindungsgemäßen Ausführung des Schraubenantriebs besteht darin, dass bei Komplikationen während einer Operation sehr rasch und sicher, mit einfachsten Mitteln, die Schrauben wieder entfernt und geeignete Korrekturen vorgenommen werden können. Dies ist bei Schrauben in anderen Einsatzgebieten ebenfalls der Fall.

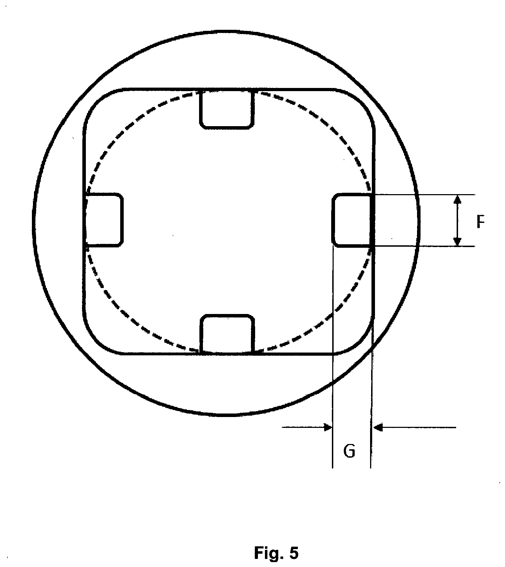

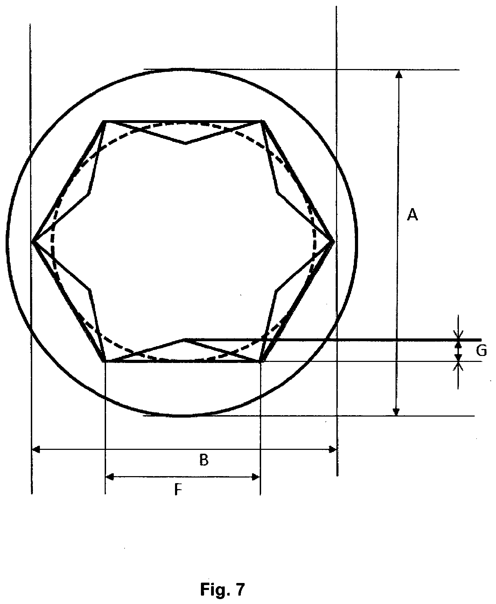

Die Ausführung der Erfindung ist nicht an einen Schraubenkopf mit Innensechskant gebunden. Die Zapfen, durch die das Drehmoment begrenzt wird, können ebenso an einen Innen-Vierkant-, -Dreikant- oder - Achtkant- Schraubenkopf an gearbeitet sein. Das Prinzip einer Ausführung mit Innenvierkant-Antrieb und angesetzten Zapfen vor und nach dem Abscheren der Zapfen bei Überbelastung der Schraube zeigen die Figuren 5 und 6. Auch ist es nicht notwendig, mit Torx Zapfen, die genau der Iso - Norm entsprechen, eine Begrenzung des Drehmoments einzustellen. Es können stattdessen auch dreieckige Zapfen (Figur 7), viereckige Zapfen (Figur 6) - mit oder ohne Fasen - halbrunde (Figur 2) oder ovale Zapfen als Überdrehsicherung verwendet werden.

Um sehr niedrige Drehmomente abzusichern, können einzelne Zapfen weg gelassen werden. So können statt der 6 Zapfen auf einem Innensechskant Profil auch nur 3 Zapfen verwendet werden.

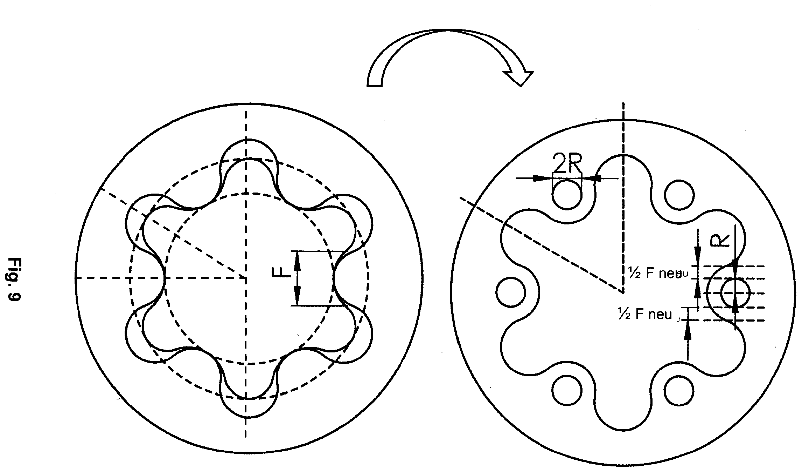

Zudem können die Zapfen noch gezielt geschwächt werden, um das maximale Drehmoment besonders fein und präzise an die Gesamtkonstruktion der Schraube anzupassen. Dies wird durch Bohrungen erreicht, die in die Zapfen eingebracht werden. Figur 9 zeigt diese Möglichkeit am Beispiel eines Innensechsrund-Schraubenkopfs. Durch Bohrungen mit Radius R wird die Dicke F der Zapfen an der Basis geschwächt. Je größer R ist, umso stärker ist die Schwächung des Zapfens. Die Dicke des Zapfens an der Basis reduziert sich dabei von F auf Fₙₑᵤ = F - 2R. Das Drehmoment, bei dem die Zapfen abbrechen, kann somit gezielt herabgesetzt werden. Mit dieser Detailkonstruktion kann die erfindungsgemäße Drehmomentsicherung an biodegradierbare Magnesiumschrauben aller Art und Abmessungen, an alle denkbaren Gewindeformen und auch an Magnesiumwerkstoffe verschiedener Festigkeiten, sowie an alle anderen Werkstoffe wie Kunststoffe, Holz, Metall oder Legierungen aller Art angepasst werden.

Die Herstellung der erfindungsgemäßen drehmomentgesicherten Schraubenantriebe erfolgt vorzugsweise durch filigranes Ausfräsen der in den Zeichnungen näher dargestellten Formen aus einem Schrauben-Rohling. Alternativ kann der Schraubenkopf mit dem erfindungsgemäßen drehmomentgesicherten Antrieb auch durch mechanisches Pressen eines Rohlings in einer entsprechenden Form oder durch das Verfahren des Druckgießens gefertigt werden. Als Werkstoff für den Rohling kann jedes gewünschte Material eingesetzt werden. Für das Gebiet der Osteosynthese kann dabei biodegradierbare Magnesiumlegierung eingesetzt werden, vorzugsweise wird man jedoch eine Legierung verwenden, die aus 1,5 bis 5 Gew. % Seltener Erden, davon 1,5 bis 2,5 Gew. % Neodym, 1,5 bis 5,0 Gew. % Yttrium, 0,01 bis 1,0 Gew. % Zink, 0.1 bis 2,5 Gew. % Zirkonium, Rest Magnesium, besteht, wobei die Gehalte an Verunreinigungen durch AI, Si, Ni und Fe unter jeweils 0,5 Gew. % liegen.

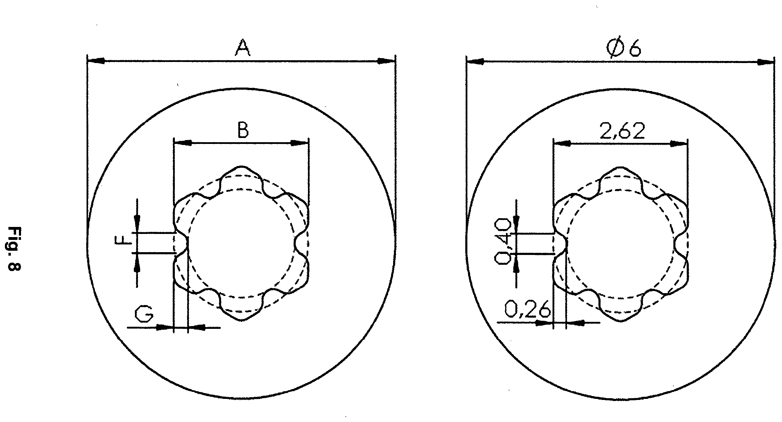

Eine besonders vorteilhafte Ausführung der Erfindung ist in Figur 8 dargestellt. Sie bezieht sich auf eine Schraube, die aus einer Magnesiumlegierung aus 1,5 bis 5 Gew. % Seltener Erden, davon 1,5 bis 2,5 Gew. % Neodym, 1,5 bis 4,0 Gew. % Yttrium, 0,01 bis 1,0 Gew. % Zink, 0.2 bis 2,0 Gew. % Zirkonium, Rest Magnesium, besteht. Abgebildet ist der drehmomentgesicherte Schraubenantrieb einer Schraube gemäß Figur 1 mit den Abmessungen A = 6,0 mm, B = 2,62 mm, C = 1,8 mm, D = 3,5 mm, E = 8,0 bis 40 mm. In den Schraubenkopf ist ein Innensechskant-Antrieb von B = 2,62 mm eingearbeitet, auf dessen Kanten Torx Zapfen der Abmessungen F = 0,40 mm und G = 0,26 mm aufgesetzt sind. Die Zapfen liegen in einer Ebene mit dem Innen-Sechskant-Antrieb und sind an dessen Kanten angesetzt. Dieser Schraubenkopf ist drehmomentgesichert bei einem Drehmoment von 1,42 +/- 0,12 Nm. Wird dieses Drehmoment überschritten, brechen die Zapfen ab und die Schraube kann keinen weiteren Schaden nehmen. Sie kann dann mit einem geeigneten Sechskant - Inbus aus dem Knochen herausgedreht und durch eine andere Schraube ersetzt werden.

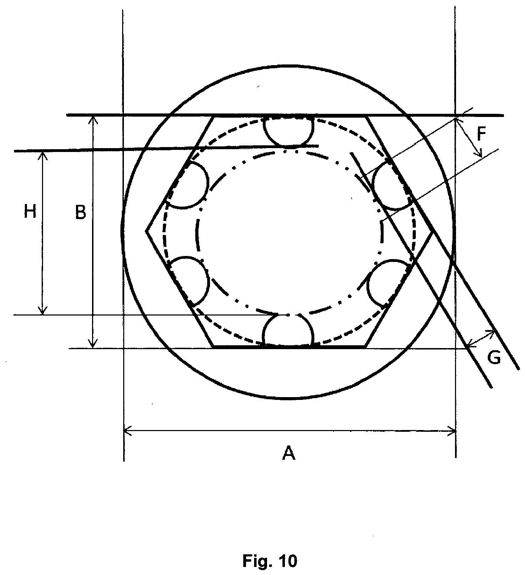

In Figur 10 ist die vorteilhafte Ausgestaltungsform dargestellt, bei der der Innen-Mehrkant-Antrieb tiefer in der Schraube gelegen als der Innen- Mehrrund-Antrieb. Die Zapfen des Innen-Mehrrund Antriebes liegen dann nicht mit den Kanten des Innen-Mehrkant-Antrieb in einer Ebene, sondern darüber.

Wie dargestellt, weist hier der Innen-Mehrkant-Antrieb einen geringeren Durchmesser weist als der Innen-Mehrrund-Antrieb. Als Durchmesser für den Innen-Mehrrund-Antrieb ist dabei der Kreis zu verstehen, der die Zapfen innen an ihrer Basis tangiert (H). Als Durchmesser für den Innen-Mehrkant-Antrieb ist dabei der Kreis zu verstehen, der alle Kanten tangiert (B).

### Bezugszeichen

- A: Durchmesser des Schraubenkopfs
- B: Durchmesser des Innen-Mehrkant Antriebs
- C: Tiefe des Schraubenantriebs
- D: Durchmesser des Schraubenschafts bzw. Gewinde-Durchmesser (außen)
- E: Gesamtlänge der Schraube
- F: Dicke der drehmomentsichernden Zapfen an der Basis
- G: Höhe der drehmomentsichernden Zapfen
- G*: Rest Höhe der Zapfen nach Abbrechen
- R: Radius der Bohrung in den Zapfen zur Schwächung des maximalen Drehmoments
- H: Durchmesser des Innen-Mehrrund-Antriebs

## Patentansprüche

1. Schraube mit Schraubenantrieb, bei dem
- in einen Schraubenkopf zwei Schraubenantriebe eingearbeitet sind, wobei ein Antrieb ein Innen-Mehrkant-Antrieb ist, und
- der zweite Antrieb ein Innen-Mehrrund -Antrieb ist, wobei
der Innen-Mehrrund-Antrieb ein Schraubenantrieb in form eines mehrstrahligen Sterns ist, bei dem die Strahlen in Form von abgerundeten Zapfen oder anderen Formen der Zapfen geformt sind,
**dadurch gekennzeichnet, dass**
- der Schraubenantrieb eine integrierte Drehmomentsicherung umfasst, und
- bei Überschreiten eines auf die Konstruktion der Schraube abgestimmten Drehmoments die den Antrieb ermöglichenden Zapfen abbrechen, abscheren oder flach gedrückt werden, so dass eine Beschädigung des Gewindes, des Schraubenschafts oder das Abreißen des Schraubenkopfs verhindert wird.

2. Schraube mit Schraubenantrieb nach Anspruch 1, **dadurch gekennzeichnet, dass** der Innen-Mehrrund-Antrieb so auf den Innen-Mehrkant-Antrieb angesetzt ist, dass nach dem Abbrechen der Zapfen des Innen-Mehrrund-Antriebs aufgrund des Überschreitens des maximalen Drehmoments der Mehrkant-Innen-Antrieb erhalten bleibt.

3. Schraube mit Schraubenantrieb nach Anspruch 1 oder 2, **dadurch gekennzeichnet, dass** der Innen-Mehrkant-Antrieb als Innen-Sechskant- Antrieb oder als Innen-Fünfkant-Antrieb oder als Innen-Vierkant-Antrieb oder als Innen-Dreikant-Antrieb ausgebildet ist.

4. Schraube mit Schraubenantrieb nach einem der vorhergehenden Ansprüche, **dadurch gekennzeichnet, dass**
der Durchmesser des Innen-Mehrkant-Antriebs (B) 1-20 mm,
die Tiefe des Schraubenantriebs (C) 0,5-6 mm,
die Dicke der drehmomentsichernden Zapfen an der Basis (F) 0,1-4 mm,
die Höhe der drehmomentsichernden Zapfen (G) 0,1-3 mm,
die Rest Höhe der Zapfen nach Abbrechen (G*) 0,01-1 mm,
und der Durchmesser der Bohrung in den Zapfen zur Schwächung des maximalen Drehmoments (2R) 0,06-1,6 mm beträgt.

5. Schraube mit Schraubenantrieb nach einem der vorhergehenden Ansprüche, **dadurch gekennzeichnet, dass**
dieser aus mindestens einem Polymer oder aus Holz oder aus Keramik oder aus Composite oder aus mindestens einem Metall besteht.

6. Schraube mit Schraubenantrieb nach einem der vorhergehenden Ansprüche,
**dadurch gekennzeichnet, dass**
eine biodegradierbare Magnesiumschraube aus einer Magnesiumlegierung mit einem Seltenerdmetallanteil von 1,5 bis 5 Gew. %, davon 1,5 bis 2,5 Gew. % Neodym, einem Yttriumanteil von 1,5 bis 5 Gew. %, einem Zirkoniumanteil von 0,1 bis 2,5 Gew. %, einem Zinkanteil von 0,01 bis 1 Gew. % sowie unvermeidbaren Verunreinigungen, besteht, wobei der Gesamtgehalt an möglichen Verunreinigungen unterhalb von 1 Gew. % liegt und der Aluminiumanteil kleiner als 0,5 Gew. % ist, und der Rest zu 100 Gew. % Magnesium enthält.

7. Schraube mit Schraubenantrieb nach einem der vorhergehenden Ansprüche, **dadurch gekennzeichnet, dass**
der Durchmesser des Schraubenkopfs (A) 2-30 mm, der
Durchmesser des Innen-Mehrkant-Antriebs (B) 1-20 mm,
die Tiefe des Schraubenantriebs (C) 0,5-6 mm,
der Durchmesser des Schraubenschafts / Gewinde-Durchmesser (D) 2-15 mm,
die Gesamtlänge der Schraube (E) 5-120 mm,
die Dicke der drehmomentsichernden Zapfen an der Basis (F) 0,1-4 mm,
die Höhe der drehmomentsichernden Zapfen (G) 0,1-3 mm,
die Rest Höhe der Zapfen nach Abbrechen (G*) 0,01-1 mm,
und der Durchmesser der Bohrung in den Zapfen zur Schwächung des maximalen Drehmoments (2R) 0,06-1,6 mm beträgt.

8. Schraube mit Schraubenantrieb nach einem der Ansprüche 1-5, 7, **dadurch gekennzeichnet, dass**
diese aus mindestens einem Polymer oder aus Holz oder aus Keramik oder aus Composite oder aus mindestens einem Metall besteht.

## Claims

1. A screw having a screw drive in which
- two screw drives are incorporated in a screw head, wherein one drive is an internal polygonal drive, and
- the second drive is an internal multilobular drive, wherein
the internal multilobular drive is a screw drive in the form of a multi-ray star, in which the rays are formed in the shape of rounded points or other shapes of the points,
**characterised in that**
- the screw drive comprises an integrated torque protection, and
- when a torque adapted to the design of the screw is exceeded, the points enabling the drive break off, shear or are pressed flat to prevent damage to the thread and the screw shaft or the tearing off of the screw head.

2. The screw having a screw drive according to claim 1, **characterised in that** the internal multilobular drive is attached to the internal polygonal drive such that, after the points of the internal multilobular drive break off on account of the exceeded maximum torque, the polygonal internal drive is preserved.

3. The screw having a screw drive according to claim 1 or 2, **characterised in that** the internal polygonal drive is designed as an internal hexagonal drive or as an internal pentagonal drive or as an internal square drive or as an internal triangular drive.

4. The screw having a screw drive according to any one of the preceding claims, **characterised in that**
the diameter of the internal polygonal drive (8) is 1-20 mm,
the depth of the screw drive (C) is 0.5-6 mm,
the thickness of the torque protection points at the base (F) is 0.1-4 mm,
the height of the torque protection points (G) is 0.1-3 mm,
the residual height of the points after breaking off (G*) is 0.01-1 mm,
and the diameter of the drilled hole in the points for weakening the maximum torque (2R) is 0.06-1.6 mm.

5. The screw having a screw drive according to any one of the preceding claims, **characterised in that**
said screw drive consists of at least one polymer, or wood, or ceramic or composite material, or at least one metal.

6. The screw having a screw drive according to any one of the preceding claims, **characterised in that**
a biodegradable magnesium screw consists of a magnesium alloy having a rare earth metal content of 1.5 to 5% by weight, of which 1.5 to 2.5% by weight is neodymium, an yttrium content of 1.5 to 5% by weight, a zirconium content of 0.1 to 2.5% by weight a zinc content of 0.01 to 1% by weight as well as unavoidable impurities, wherein the total content of possible impurities is below 1% by weight and the aluminium content is less than 0.5% by weight, and the balance up to 100% is magnesium.

7. The screw having a screw drive according to any one of the preceding claims, **characterised in that**
the diameter of the screw head (A) is 2-30 mm, the
diameter of the internal polygonal drive (B) is 1-20 mm,
the depth of the screw drive (C) is 0.5-6 mm,
the diameter of the screw shaft/thread diameter (D) is 2-15 mm,
the total length of the screw (E) is 5-120 mm,
the thickness of the torque protection points at the base (F) is 0.1-4 mm,
the height of the torque protection points (G) is 0.1-3 mm,
the residual height of the points after breaking off (G*) is 0.01-1 mm,
and the diameter of the drilled hole in the points for weakening the maximum torque (2R) is 0.06-1.6 mm.

8. The screw having a screw drive according to any one of claims 1-5 and 7, **characterised in that**
said screw consists of at least one polymer, or wood, or ceramic or composite material, or at least one metal.

## Revendications

1. Vis avec entraînement de vis, dans laquelle
- deux entraînement de vis sont incorporées dans une tête de vis, l'une d'elles étant un entraînement polygonal creux, et
- le deuxième entraînement est un entraînement creux multi-rondes, où l'entraînement creux multi-rondes est un entraînement de vis en forme d'étoile multiradiale, dans lequel les rayons sont formés en forme de cônes arrondis ou d'autres formes de cônes,
**caractérisé en ce que**
- l'entraînement de vis comporte un dispositif de blocage de torque intégré, et
- si un torque adapté à la construction de la vis est dépassé, les cônes permettant l'entraînement sont rompus, cisaillées ou aplaties afin d'éviter d'endommager le filetage, la tige ou l'arrachement de la tête de la vis.

2. Vis avec entraînement de vis selon la revendication 1, **caractérisée en ce que** l'entraînement multi-rondes creux est placé sur l'entraînement polygonal creux de telle sorte
**que**, après que les cônes de l'entraînement multi-rondes creux se sont rompus en raison du dépassement du torque maximal, l'entraînement polygonal creux est conservé.

3. Vis avec entraînement de vis selon la revendication 1 ou 2, **caractérisée en ce que** l'entraînement polygonal creux est conçu comme entraînement hexagonal creux ou comme
entraînement pentagonal creux ou comme entraînement carré creux ou comme entraînement triangulaire creux.

4. Vis avec entraînement de vis selon l'une des revendications précédentes, **caractérisée en ce que**
le diamètre de l'entraînement polygonal creux (B) est de 1-20 mm,
la profondeur de l'entraînement de vis (C) est de 0,5-6 mm,
l'épaisseur des cônes de blocage du torque (F) est de 0,1-4 mm à la base,
la hauteur des cônes de blocage du torque (G) est de 0,1-3 mm,
la hauteur restante des cônes après rupture (G*) est de 0,01-1 mm,
et le diamètre du trou dans les cônes pour affaiblir le torque maximal (2R) est de 0,06-1,6 mm.

5. Vis avec entraînement de vis selon l'une des revendications précédentes, **caractérisée en ce que**
il est constitué d'au moins un polymère ou de bois ou de la céramique ou du composite ou
d'au moins un métal.

6. Vis avec entraînement de vis selon l'une des revendications précédentes,
**caractérisé par**
une vis biodégradable en alliage de magnésium avec
une teneur en métaux des terres rares de 1,5 à 5 % en poids,
dont 1,5 à 2,5 % en poids de néodyme,
une teneur en yttrium de 1,5 à 5 % en poids,
une teneur en zirconium de 0,1 à 2,5 % en poids,
une teneur en zinc de 0,01 à 1 % en poids et
les impuretés inévitables, la teneur totale des impuretés éventuelles étant inférieure à 1 % en poids et
la teneur en aluminium étant inférieure à 0,5 % en poids,
le reste jusqu'à 100 % en poids étant du magnésium.

7. Vis avec entraînement de vis selon l'une des revendications précédentes, **caractérisée en ce que**
le diamètre de la tête de vis (A) est de 2-30 mm,
le diamètre de l'entraînement polygonal creux (B) est de 1-20 mm,
la profondeur de la vis (C) est de 0,5-6 mm,
le diamètre de la tige de la vis / du filetage (D) est de 2-15 mm,
la longueur totale de la vis (E) est de 5-120 mm,
l'épaisseur des cônes de blocage du torque à la base (F) est de 0,1-4 mm,
la hauteur des cônes de blocage du torque (G) est de 0,1-3 mm,
la hauteur restante des cônes après rupture (G*) est de 0,01-1 mm,
et le diamètre du trou dans les cônes pour affaiblir le torque maximal (2R) est de 0,06-1,6 mm.

8. Vis avec entraînement de vis selon l'une des revendications 1 -5, 7, **caractérisée en ce que**
il est constitué d'au moins un polymère ou de bois ou de céramique ou de composite ou d'au moins un métal.
